# EUROPEAN PATENT SPECIFICATION

(11) **EP 3 897 440 B1**
(45) Date of publication and mention of the grant of the patent: **29.04.2026**
(21) Application number: 19900525.7
(22) Date of filing: 22.11.2019
(51) Int. Cl.: A61B 34/30, A61B 34/00, A61B 17/34, A61B 90/50, A61B 17/00, A61B 34/20, A61B 90/00

(54) **ROBOTIC SURGICAL SYSTEMS HAVING ROBOTIC ARM ASSEMBLIES**
ROBOTISCHE CHIRURGISCHE SYSTEME MIT ROBOTERARMANORDNUNGEN
SYSTÈMES CHIRURGICAUX ROBOTIQUES MUNIS D'ENSEMBLES DE BRAS ROBOTIQUE

(30) Priority: 17.12.2018 US 201862780358 P
(43) Date of publication of application: 27.10.2021
(73) Proprietor: Covidien LP, Mansfield, MA 02048 (US)
(72) Inventor: KAPADIA, Jaimeen, Cambridge, Massachusetts 02139 (US)
(74) Representative: Zimmermann & Partner Patentanwälte mbB
(86) International application number: PCT/US2019/062840
(87) International publication number: WO 2020/131304

(56) References cited:
- WO-A1-2015/167808
- WO-A1-2017/205333
- WO-A1-2017/205333
- WO-A1-2019/075571
- KR-A- 20180 083 306
- US-A1- 2008 065 111
- US-A1- 2018 049 824
- US-A1- 2018 153 634
- US-A1- 2018 168 748
- US-A1- 2018 177 557

## Description

### BACKGROUND

Robotic surgical systems have been used in minimally invasive medical procedures and can include robotic arm assemblies. Some robotic arm assemblies include a robot arm having an instrument drive assembly coupled thereto for coupling surgical instruments to the robot arm, such as, for example, a pair of jaw members, electrosurgical forceps, cutting instruments, or any other endoscopic or open surgical devices, and a mounting arm coupled thereto for coupling surgical accessories to the robot arm, such as, for example, a surgical port such as a trocar.

Prior to or during use of the robotic surgical system, surgical instruments are selected and connected to the instrument drive assembly of each robot arm, where the instrument drive assembly can drive the actuation of an end effector of the surgical instrument. Such instrument drive assemblies can include many components that add to the cost, size, and energy output of the robotic surgical system.

Under certain procedures, a surgical accessory, such as, for example, a surgical port may be docked or undocked to/from a mounting arm of the robot arm by clamping or unclamping steps. During a procedure, the end effector and/or a portion of the surgical instrument may be inserted through the surgical port, and a small incision or a natural orifice of a patient, to bring the end effector proximate a working site within the body of the patient. Although such surgical ports may provide additional stability and act as a guide channel for the surgical instrument during insertion and actuation of the end effector, the clamping/unclamping steps can prolong or otherwise add to the complexity of the surgical procedure. Documents US 2018/153634 A1 and WO 2017/205333 A1 relate to surgical robotic instrument drive assemblies.

### SUMMARY

Accordingly, the present disclosure describes robotic arm assemblies having a mounting arm that advantageously provides quick and easy connection between a robot arm and a variety of surgical accessories, such as a surgical port. The present disclosure also describes robotic arm assemblies having a small, lightweight instrument drive unit that reduces the complexity of the robotic arm assemblies. The presently disclosed instrument drive unit consolidates five motors into a single unit that shares mounting features and maximizes thermal mass and heat dissipation. Advantageously, the presently disclosed instrument drive unit has a reduced height, simplifies motor mounting, provides a shorter drivetrain, reduces weight, provides a higher power density, reduces overall height of the surgical robot, reduces electronics, provides lower COGS, improves reliability, and reduces manufacturing costs.

According to an aspect of the present disclosure, a robotic arm assembly is provided. The robotic arm assembly includes a robotic arm and an instrument drive unit. The instrument drive unit is configured to provide five degrees of rotational freedom and is coupled to the robotic arm. The instrument drive unit includes a motor housing, motors supported in the motor housing and rotatable with the motor housing, and gear box assemblies coupled to the motors and configured to transmit drive power from the motors to a surgical instrument. In embodiments, the instrument drive unit may include one or more torque sensors that rotate with one or more output shafts of the motors to measure torque as drive power is transmitted form the motors to the surgical instrument.

In some embodiments, the robotic arm assembly may further include the surgical instrument.

In certain embodiments, the robotic arm assembly may further include a sterile interface module that couples the instrument drive unit to the surgical instrument.

In embodiment, the motors may include four or more motors, each motor providing one of the five degrees of rotational freedom of the instrument drive unit. In certain embodiments, the motors may include five (5) motors, four (4) motors of which drive the surgical instrument, and one (1) motor of which rotates the four (4) motors about an instrument axis. The instrument drive unit may define a longitudinal axis and the four motors may be arranged in a circle about the longitudinal axis. The motor housing and the four motors may be configured rotate together about the longitudinal axis. A first motor of the four motors may define a first motor axis. The first motor may be configured to independently rotate about the first motor axis when the motor housing rotates about the longitudinal axis of the instrument drive unit. The first motor axis of the first motor and the longitudinal axis of the instrument drive unit may be parallel to one another.

In some embodiments, a first gear box assembly of the gear box assemblies may include a reduction gear assembly and a coupler secured to the reduction gear assembly. The first motor may include a drive shaft, wherein the reduction gear assembly of the first gear box assembly couples to the drive shaft of the first motor. The reduction gear assembly may be configured to reduce an output from the drive shaft of the first motor to the coupler of the first gear box assembly.

In certain embodiments, the robotic arm assembly may further comprise a motor winding and a magnet supported about the motor housing. The motor winding may be configured to rotate with the motor housing relative to the magnet to act as a motor and provide one of the five degrees of rotational freedom.

According to yet another aspect of the present disclosure, a robotic arm assembly comprises a robotic arm, a surgical port, a sterile interface module, a surgical instrument, and an instrument drive unit. The robotic arm includes a mounting arm that extends distally to an annulus. The surgical port is coupled to the annulus. The surgical instrument is coupled to the sterile interface module. The instrument drive unit is coupled to the sterile interface module and slidably movable along the robotic arm to translate the surgical instrument along the robotic arm and selectively advance the surgical instrument through the surgical port.

In some embodiments, the annulus may be an enclosed ring defining a central opening therethrough.

In certain embodiments, the annulus may be configured to couple the surgical port to the annulus by bottom-loading. The annulus may include an inner surface and the surgical port may include a housing and a cannula that extends distally from the housing. The annulus and the housing of the surgical port may be threadably coupled.

In embodiments, the instrument drive unit may include a motor housing, motors supported in the motor housing and rotatable with the motor housing, and gear box assemblies coupled to the motors and configured to transmit drive power from the motors to the surgical instrument. The motors may include four motors. The instrument drive unit may define a longitudinal axis and the four motors may be arranged in a circle about the longitudinal axis. The motor housing and the four motors may be configured rotate together about the longitudinal axis. A first motor of the four motors may define a first motor axis. The first motor may be configured to independently rotate about the first motor axis when the motor housing rotates about the longitudinal axis of the instrument drive unit.

In certain embodiments, the robotic arm assembly may further include a motor winding and a magnet supported about the motor housing. The motor winding may be configured to rotate with the motor housing relative to the magnet.

Other aspects, features, and advantages will be apparent from the description, the drawings, and the claims that follow.

### BRIEF DESCRIPTION OF THE DRAWINGS

Embodiments of the present disclosure are described herein with reference to the accompanying drawings, wherein:
FIG. 1 is a perspective view of a robotic assembly in accordance with the present disclosure;
FIG. 2 is an enlarged, perspective view of the indicated area of detail shown in FIG. 1 and illustrating a surgical port secured to a mounting arm of the robotic arm assembly of FIG. 1;
FIG. 3 is a perspective view of the indicated area in FIG. 2 with the surgical port shown separated from the mounting arm;
FIG. 4 is an enlarged perspective view of the indicated area of detail shown in FIG. 1;
FIG. 5 is a bottom, perspective view of an instrument drive unit ("IDU") of the robotic arm assembly of FIG. 1;
FIG. 6 is a top, perspective view of the IDU of FIG. 5;
FIG. 7 is an enlarged, perspective view of the IDU of FIGS. 5 and 6 with portions thereof removed or shown in phantom for clarity;
FIG. 8 is a perspective view, with parts separated, of the IDU of FIGS. 5 and 6;
FIG. 9 is a cross, sectional view of the IDU of FIGS. 5 and 6, as taken along section line 9-9 shown in FIG. 7; and
FIG. 10 is a bottom view of the IDU of FIGS. 5 and 6 illustrating rotational movement of components of the IDU.

### DETAILED DESCRIPTION

The invention is defined in appended claim 1. Further embodiments are defined in appended dependent claims.

Embodiments of the presently disclosed robotic arm assembly are described in detail with reference to the drawings, in which like reference numerals designate identical or corresponding elements in each of the several views. As commonly known, the term "clinician" refers to a doctor, a nurse, or any other care provider and may include support personnel. Further, as is used in the art, the term "distal" refers to a position, a direction, and/or a structure, which is farther from the user, and the term "proximal" refers to a position, a direction, and/or a structure, which is closer to the user. In addition, directional terms such as front, rear, upper, lower, top, bottom, and the like are used simply for convenience of description and are not intended to limit the disclosure attached hereto.

In the following description, well-known functions or constructions are not described in detail to avoid obscuring the present disclosure in unnecessary detail.

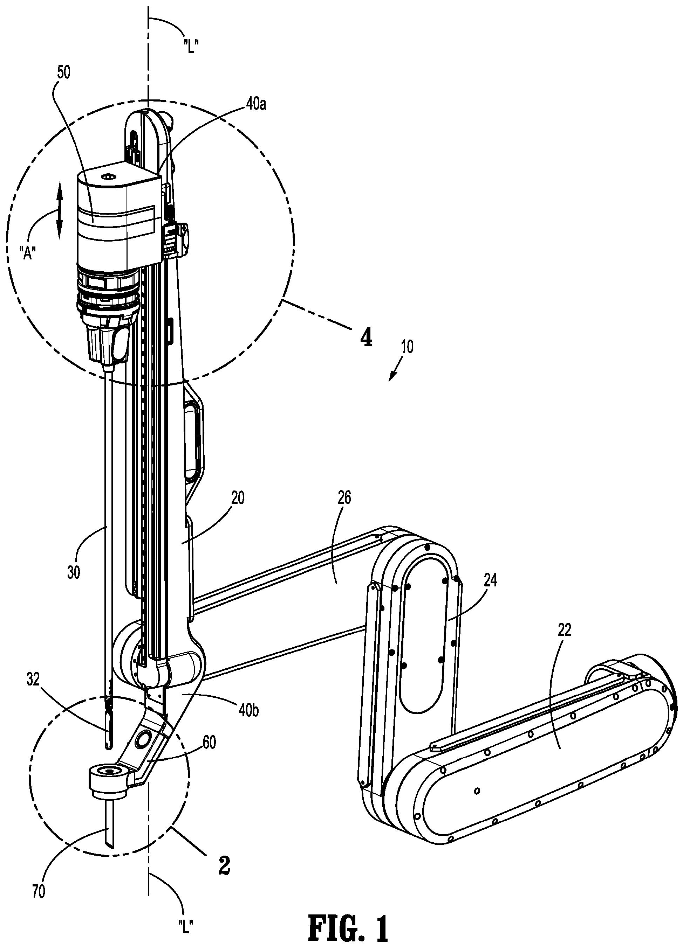

With brief reference to FIG. 1, a robotic surgical system 10 includes a robotic arm assembly 20 that supports a surgical instrument 30 having an end effector 32 (e.g., grasper, clip applier, stapler, vessel sealer, tack applier, etc.). For a more detailed description of an example surgical instrument, reference can be made to U.S. Patent Application Publication No. U.S. 2017/0224367 to Kapadia, and PCT Application Publication No. WO/2017/205310 to Zemlok et al.,

Robotic surgical system 10 employ various robotic elements to assist the clinician and allow remote operation (or partial remote operation) of surgical instrumentation such as surgical instrument 30. Various robotic arms, gears, cams, pulleys, electric and mechanical motors, etc. may be employed for this purpose and may be designed with robotic surgical system 10 to assist the clinician during the course of an operation or treatment. Such robotic systems may include remotely steerable systems, automatically flexible surgical systems, remotely flexible surgical systems, remotely articulating surgical systems, wireless surgical systems, modular or selectively configurable remotely operated surgical systems, etc.

Robotic surgical system 10 includes a medical work station (not shown) that may be employed with one or more consoles positioned next to the operating theater or located in a remote location. In this instance, one team of clinicians may prep the patient for surgery and configure robotic surgical system 10 with surgical instrument 30 while another clinician (or group of clinicians) remotely controls surgical instrument 30 via robotic surgical system 10. As can be appreciated, a highly skilled clinician may perform multiple operations in multiple locations without leaving his/her remote console which can be both economically advantageous and a benefit to the patient or a series of patients. For a detailed description of exemplary medical work stations and/or components thereof, reference may be made to U.S. Patent Application Publication No. 2012/0116416 and PCT Application Publication No. WO2016/025132.

With continued reference to FIG. 1, robotic arm assembly 20 of robotic surgical system 10 includes robotic arms 22, 24, 26. Robotic arms 22, 24, 26 of robotic arm assembly 20 are pivotally coupled together and movable together and/or relative to one another. Robotic arm 26 is coupled to a slide rail 40 that supports an IDU 50 for operating surgical instrument 30. Slide rail 40 defines a longitudinal axis "L" and includes a proximal end portion 40a and a distal end portion 40b through which longitudinal axis "L" extends. IDU 50 is slidably supported on slide rail 40 and selectively axially movable along longitudinal axis "L," as indicated by arrows "A," between a proximal position, adjacent proximal end portion 40a of slide rail 40, and distal position, adjacent distal end portions 40b of slide rail 40.

Slide rail 40 of robotic arm assembly 20 further includes a mounting arm 60 secured to distal end portion 40b of slide rail 40. Mounting arm 60 supports a surgical portal 70 for advancing surgical instrument 30 through surgical portion 70 to selectively access a surgical site. Mounting arm 60 is releasably attachable to slide rail 40. For more detailed description of how mounting arm 60 releasably attaches to slide rail 40, reference can be made to a similar mounting arm disclosed in U.S. Provisional Application Serial No. 62/613,601, filed January 4, 2018.

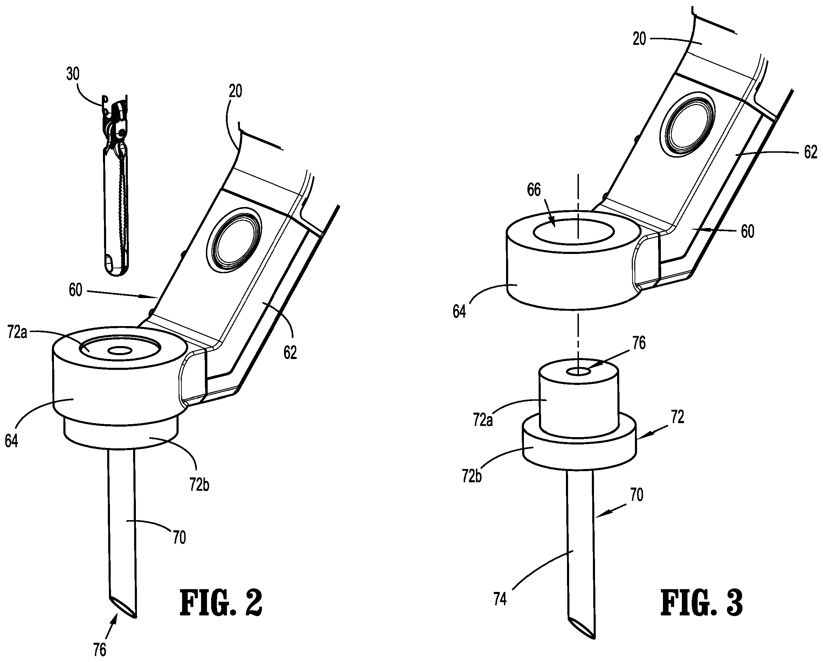

Turning now to FIGS. 2 and 3, mounting arm 60 of robotic arm assembly 20 includes a housing 62 that extends distally to an annulus 64. Annulus 64 defines a central opening 66 therethrough that is configured to receive surgical port 70 therein by bottom-loading. Surgical port 70 includes a housing assembly 72 and a cannula 74 that extends distally from housing assembly 72. Housing assembly 72 includes an upper housing 72a and a lower housing 72b. Upper housing 72a is configured to be received into annulus 64, by bottom-loading, such that annulus 64 of mounting arm 60 secures upper housing 72a within central opening 66 of annulus 64 by any suitable mechanical connection such as friction fit, snap-fit, magnetic, undercut, or the like. Lower housing 72b extends radially outward beyond upper housing 72a so as to act as an abutment or stop that contacts a bottom surface of annulus 64 to prevent housing assembly 72 of surgical port 70 from being advanced through annulus 64 of mounting arm 60. Surgical port 70 further defines central passage 76 that extends through housing assembly 72 and cannula 74 to provide surgical access for surgical instrument 30 through surgical port 70. For example, surgical port 70 may provide access to a surgical site such as the peritoneal cavity for effectuating a laparoscopic procedure with surgical instrument 30.

In some embodiments, an outer surface of upper housing 72a and an inner surface of annulus 64 may be threaded to enable housing assembly 72 of surgical port 70 to be threadably secured to annulus 64 of mounting arm 60.

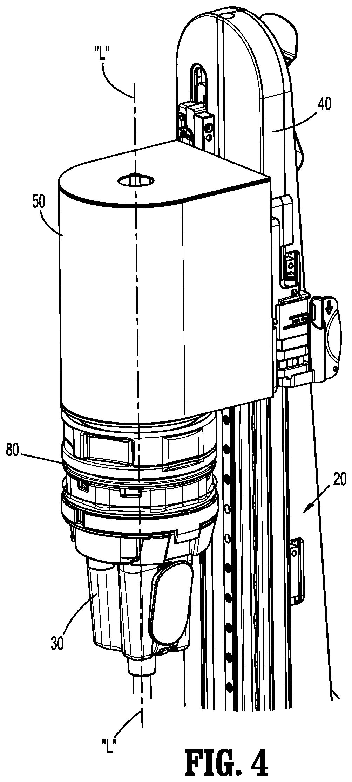

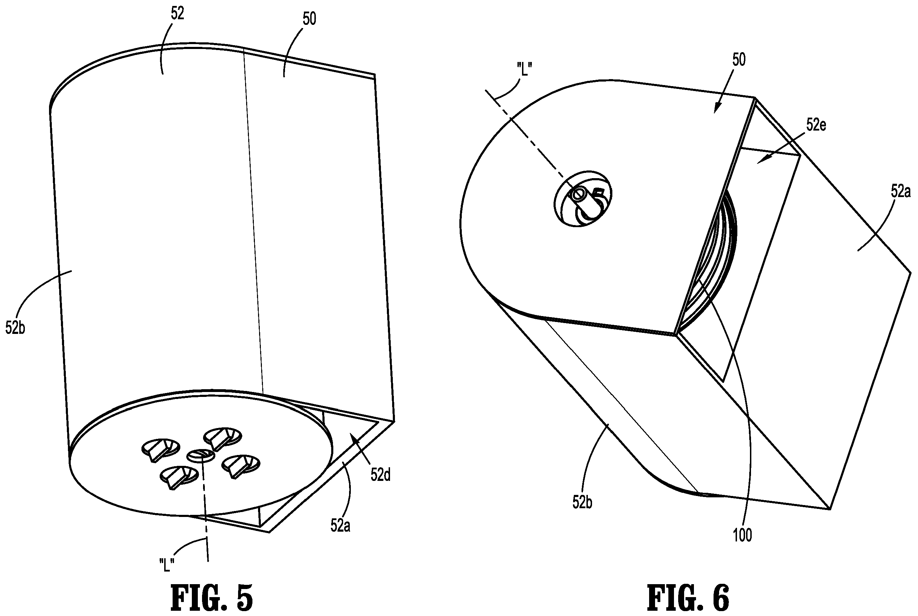

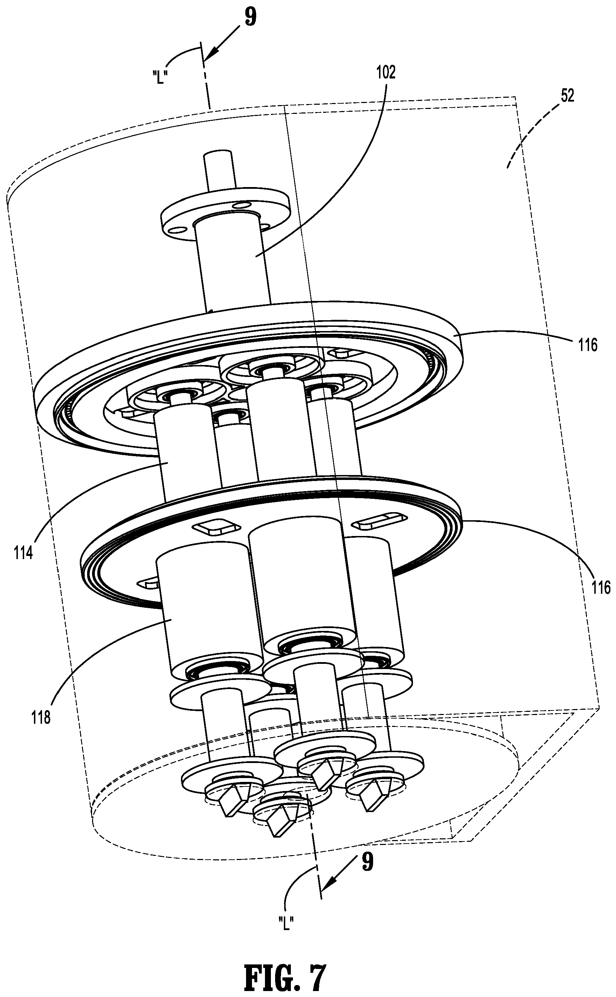

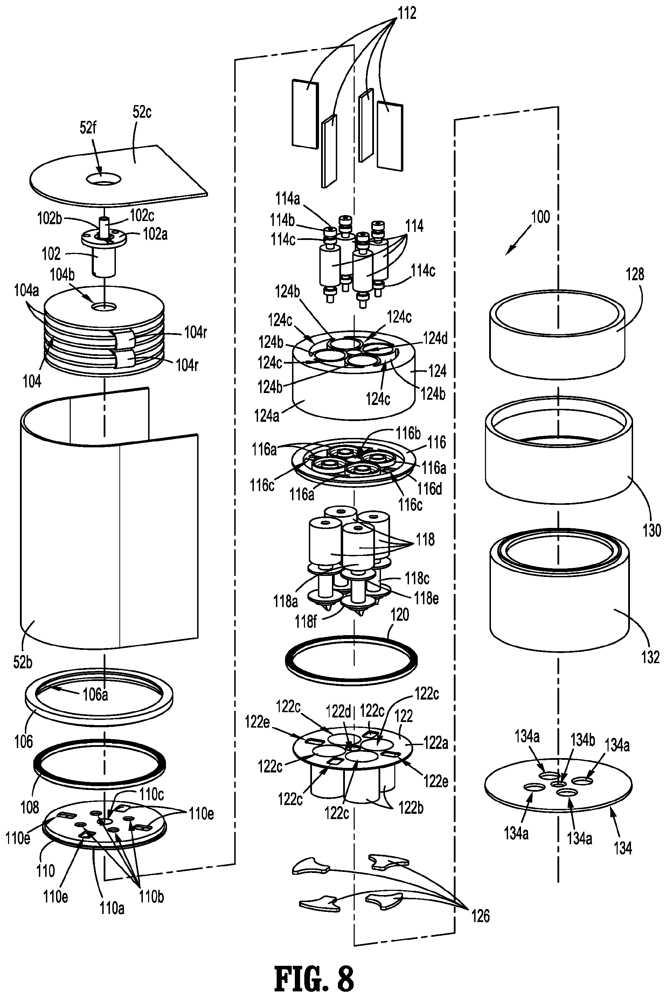

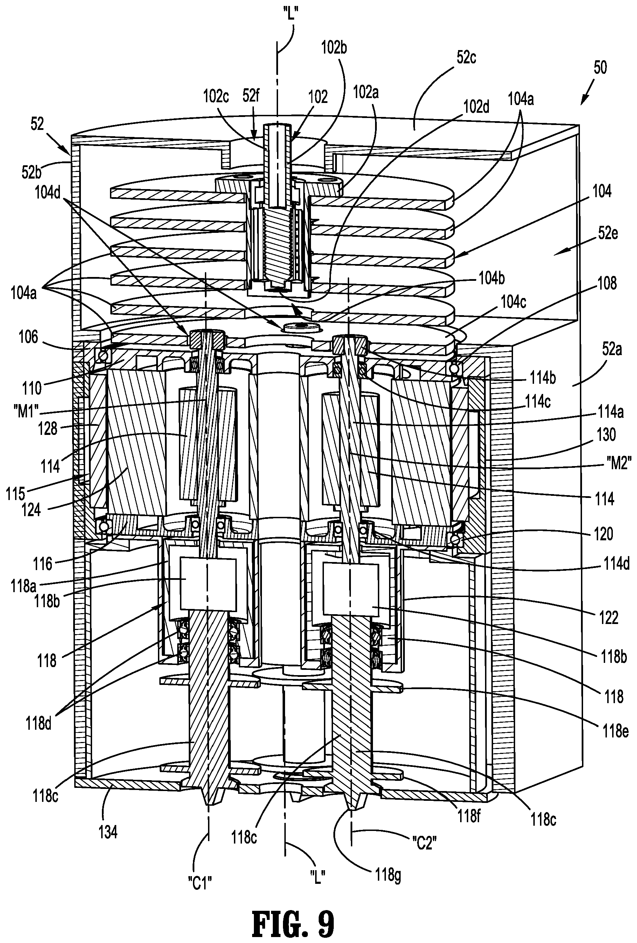

Turning now to FIGS. 4-10, IDU 50 of robotic arm assembly 20 couples to a sterile interface module 80 that interconnects IDU 50 and surgical instrument 30. For a more detailed description of sterile interface module 80, reference can be made to PCT Application Publication No. WO2016/025132. IDU 50 defines a central longitudinal axis "L" and includes an outer housing 52 having a back wall 52a, which may be planar, an arcuate front wall 52b that extends from back wall 52a, and a cover 52c supported on a top surface of front wall 52b. Outer housing 52 further defines a lower cavity 52d and an upper cavity 52e that support internal components 100 of IDU 50 and facilitate air flow through IDU 50 for cooling internal components 100 of IDU 50. Lower cavity 52d opens through a bottom surface of outer housing 52 and upper cavity 52e opens through back wall 52a of outer housing 52. Outer housing 52 further defines a central opening 52f through cover 52c.

IDU 50 of robotic arm assembly 20 includes internal components 100 arranged with integrated electronics and motors (e.g., in the form of a cluster motor) to maximize output per volume and weight. Internal components 100 of IDU 50 include a slip ring 102, electronic disc assembly 104 (e.g., magnetic and/or optical encoders), upper ring 106, upper ring bearing 108, upper plate 110, electronic boards 112, motors 114, lower plate 116, gear box assemblies 118, lower ring bearing 120, gear box housing 122, motor housing 124, torque sensors 126, magnet 128, magnet housing 130, motor winding 132, and base 134.

Slip ring 102 of IDU 50 is secured to electronic disc assembly 104 in upper cavity 52e of outer housing 52 and is configured to transfer power and/or data by electrical signals to/from IDU 50 and/or surgical instrument 30 (and/or sterile interface module 80 coupled between IDU 50 and surgical instrument 30). Slip ring 102 includes a flanged tube 102a that mounts to electronic disc assembly 104 and an inner shaft 102b about which flanged tube 102a rotates. Inner shaft 102b couples to electrical wiring (not shown) for providing electrical communication to/from surgical instrument 30 through IDU 50. The electrical wiring, which can electrically couple one or more of the presently disclosed electrical components together, can support drive and/or encoder electronics. Inner shaft 102b has a proximal portion 102c that connects to a first cable (not shown) for communicating power and/or data to/from console of robotic surgical system 10, and a distal portion 102d that connects to a second cable (not shown) for communicating power and/or data through IDU 50 and to/from surgical instrument 30 (and/or sterile interface module 80).

Electronic disc assembly 104 of IDU 50 is supported in upper cavity 52e of outer housing 52 and includes a plurality of circuit discs 104a that are coupled together by ribbons 104r and folded in a stacked and spaced-apart relation to one another. The plurality of circuit discs 104a defines a central lumen 104b therethrough that receives slip ring 102 therein so that flanged tube 102a of slip disc 102 secures to the plurality of circuit discs 104a. The plurality of circuit discs 104a includes a bottom circuit disc 104c that defines a plurality of apertures 104e therethrough. The plurality of apertures 104d of bottom circuit disc 104c align with motors 114 (e.g., four apertures, each aperture circumferentially spaced, for example, 90 degrees out-of-phase from adjacent apertures).

Upper ring 106 of IDU 50 is mounted to outer housing 52 and defines an annular channel 106a that supports upper ring bearing 108 therein. Upper ring bearing 108 is coupled to upper plate 110 for rotatably supporting upper plate 110 relative to upper ring 106. Upper plate 110 includes an outer ledge 110a that supports upper ring bearing 108 on an outer surface of upper plate 110. Upper plate 110 further defines a plurality of annular openings 110b therethrough that aligns with motors 114. Upper plate 110 also defines a central aperture 110c and a plurality of cable apertures 110e that are configured to receive electrical wiring therethrough (e.g., from slip ring 102 and/or torque sensors 126) and to facilitate air flow through IDU 50.

Motor housing 124 of IDU 50, which may be formed of any suitable iron-based and/or soft magnetic material), supports upper ring 106 and includes an outer ring 124a that supports a plurality of sleeves 124b. Each sleeve 124b is configured to function as a motor stator for a respective one of the motors 114. Sleeves 124b are positioned to receive motors 114 therein. Motor housing 124 defines a plurality of elongated channels 124c between adjacent sleeves 124b. Elongated channels 124c are positioned to support electronic boards 112 (and/or electrical wiring) therein and facilitate air flow therethrough. Motor housing 124 further defines a central aperture 124d that is configured to receive electrical wiring therethrough (e.g., from slip ring 102). Motor housing 124 is configured to rotate about central axis "L" of IDU 50 with motor winding 132, upper plate 110, lower plate 116, motors 114, electronic boards 112, electronic disc assembly 104, and flanged tube 102a of slip ring 102. Motor winding 132of IDU 50, which includes any number and/or arrangement of conductive coils (not shown) therein, is positioned to surround motor housing 124 and is secured to motor housing 124 for rotating with motor housing 124 about central axis "L" of IDU 50 relative to magnet 128. Rotation of motor winding 132 about central axis "L" functions as another motor 115 of IDU 50 (e.g., the 5^{th} motor where the four motors 114 constitute the first four motors such that IDU 50 provides five (5) degrees of freedom).

Motor winding 132 is disposed between an outer surface of outer ring 124a of motor housing 124 and an inner surface of magnet 128. Magnet 128 is fixedly secured to motor housing 128, and motor housing 128 is fixedly secured to outer housing 52 of IDU 50 (e.g., bonded).

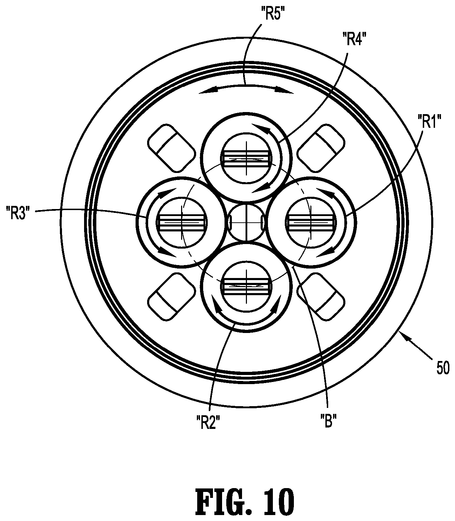

Lower plate 116 of IDU 50 supports motor housing 124 and includes a plurality of stands 116a that rotatably support motors 114 in a bolt circle "B" (e.g., 25.6mm diameter) (see FIG. 10). Stands 116a are aligned with sleeves 124b of motor housing 124. Lower plate 116 further defines a central aperture 116b and a plurality of cable apertures 116c that are configured to receive electrical wiring therethrough (e.g., from slip ring 102 and/or torque sensors 126) and to facilitate air flow through IDU 50. Lower plate 116 further includes a side wall 116d that supports lower ring bearing 120. Lower ring bearing 120 is positioned between side wall 116d of lower plate 116 and a lower portion of magnet housing 130 to enable rotational movement of lower plate 116 about central axis "L" of IDU 50.

Motors 114 (e.g., four motors) of IDU 50 include drive shafts 114a that extend between upper and lower plates 110, 116 of IDU 50. Proximal end portions of drive shafts 114a are coupled to the plurality of apertures 104d of bottom circuit disc 104c by disc bearings 114b. Drive shafts 114a further support shaft bearings 114c that enable drive shafts 114a to rotate within sleeves 124b of motor housing 124. Distal end portions of drive shafts 114a of motors 114 are coupled to gear box assemblies 118. Each motor 114 defines its own longitudinal axis (e.g., motor axes "M1," "M2," etc.) about which the respective drive shafts 114a are configured to rotate (e.g., independently and/or with the other motors 114).

Each gear box assembly 118 of IDU 50 includes a gear box 118a, a reduction gear assembly 118b (e.g., planetary or harmonic drive gear box) supported in gear box 118a, and a coupler 118c.

Gear box 118a of gear box assembly 118 is seated in, and secured to, gear box housing 122 of gear box assembly 118. Gear box 118 supports reduction gear assembly 118b between a distal end portion of drive shaft 114a of a respective one of motors 114 and a proximal end portion of coupler 118c. Reduction gear assembly 118b can include any number and/or arrangement of gears sufficient to reduce rotational output from motor 114 to coupler 118c of gear box assembly 118 for effectuating desired rotational output of coupler 118c as motor 114 rotates, regardless of whether respective couplers 118c and motors 114 are configured with aligned or misaligned arrangements. With reduction gear assembly 118b capable of having any suitable configuration, couplers 118c can be aligned with and/or offset from respective motors 114 as desired. Each gear box assembly 118 further includes coupler bearings 118d that facilitate rotational movement of couplers 118c relative to gear box 118a.

Each coupler 118c of gear box assemblies 118 defines its own longitudinal axis (e.g., coupler axes "C1," "C2," etc.) about which the respective coupler 118c rotates. Couplers 118c include an upper flange 118e and a lower flange 118f that can be encoded to facilitate torque sensing with torque sensors 126 of IDU 50. For example, torque sensors 126 can be configured to determine deflection differences between upper and lower flanges 118e, 118f as coupler 118c rotates about its respective coupler axis for measuring and/or comparing torque. Torque may also be measured by sensing and/or analyzing data indicating variation in field lines due to stress. Couplers 118c further include a drive tip 118g that is configured to engage a drive assembly (not shown) of sterile interface module 80 (FIG. 4) for operating and/or manipulating surgical instrument 30 secured to sterile interface module 80. For a more detailed description of such drive assembly and such engagement/operation of surgical instrument 30, reference can be made to PCT Application Publication No. WO2016/025132.

Gear box housing 122 of gear box assembly 118 includes a platform 122a and a plurality of gear box casings 122b that extends distally from platform 122a. Gear box housing 122 defines a bores 122c that extend through platform 122a and into gear box casings 122b for receiving gear boxes 118a of gear box assemblies 118 therein. Gear box housing 122 also defines a central aperture 122d and a plurality of cable apertures 122e that are configured to receive electrical wiring therethrough (e.g., from slip ring 102 and/or torque sensors 126) and to facilitate air flow through IDU 50.

Base 134 of IDU 50 couples to motor winding 132 of IDU 50 and encloses internal components 100 of IDU 50 within outer housing 52 of IDU 50. Base 134 defines a plurality of coupler openings 134a configured to receive drive tips 118g of couplers 118c therethrough. Base 134 further defines a central opening 134b configured to receive electrical wiring therethrough (e.g., from slip ring 102) and to facilitate air flow through IDU 50.

With reference to FIGS. 1-10, in use, surgical port 70 of robotic arm assembly 20 is attached to mounting arm 60 of robotic arm assembly 20 and surgical instrument 30 of robotic arm assembly 20 is attached to IDU 50 of robotic arm assembly 20 so that end effector 32 of surgical instrument 30 can be advanced through surgical port 70 for accessing a target site within a patient. One or more of motors 114, 115 of IDU 50 can be actuated, as indicated by arrows "R1"-"R5" (FIG. 10), independently and/or simultaneously to operate (e.g. fire) and/or manipulate (e.g., articulate, rotate, translate, etc.) surgical instrument 30 (and/or end effector 32 thereof).

In embodiments, IDU 50 of robotic arm assembly 20 may have the following specifications:

### 4X Motor Specification

| | |
|---|---|
| No Load Speed: | 1400 RPM (Min) |
| Continuous Torque: | 350 Nmm |
| Stall Torque: | 1000 Nmm |

### Operating Conditions

| | |
|---|---|
| 10% Duty Cycle: | 500 Nmm @ 500 RPM |
| 25% Duty Cycle: | 350 Nmm @ 1300 RPM |
| Max permissible volume | Cylinder 60 mm diameter and 100 mm height |
| Coupler spacing | 25.6 mm x 4 |
| Coupler material | 17-4 SS, 30 HRC min preferred |

### 5th Motor Specification

| | |
|---|---|
| No Load Speed: | 250 RPM (Min) |
| Continuous Torque: | 500 Nmm |
| Stall Torque: | 2500 Nmm |

### Operating Conditions

| | |
|---|---|
| 5% Duty Cycle: | 1000 Nmm @ 100 RPM |
| 50% Duty Cycle: | 500 Nmm @ 100 RPM |

### General Specifications

| | |
|---|---|
| Nominal Voltage: | 24/48 VDC |
| Encoder-Incremental Position Motor Side: | 2000 Cnts/Rev (Min) |
| Minimum Service Life: | 10k Hours of Operation |
| Operating Temperature Range: | 10 to 60 Degrees Centigrade |
| Integrated Torque Sensing: | +/-0.5% |

As can be appreciated, securement of any of the components of the presently disclosed apparatus can be effectuated using known securement techniques such welding, crimping, gluing, fastening, etc.

Persons skilled in the art will understand that the structures and methods specifically described herein and illustrated in the accompanying figures are non-limiting exemplary embodiments, and that the description, disclosure, and figures should be construed merely as exemplary of particular embodiments.

## Claims

1. A robotic arm assembly (20) comprising:
a robotic arm (22, 24, 26);
an instrument drive unit (50) configured to provide five degrees of rotational freedom coupled to the robotic arm and including:
a motor housing (124);
motors (114) supported in the motor housing and rotatable with the motor housing, wherein the motors include four motors, each motor providing one of the five degrees of rotational freedom of the instrument drive unit;
a motor winding (132) and a magnet (128) positioned to surround the motor housing,
the motor winding configured to rotate with the motor housing relative to the magnet to act as a motor and provide one of the five degrees of rotational freedom; and
gear box assemblies (118) coupled to the motors and configured to transmit drive power from the motors to a surgical instrument (30).

2. The robotic arm assembly of claim 1, further comprising the surgical instrument.

3. The robotic arm assembly of claim 2, further comprising a sterile interface module (80) that couples the instrument drive unit to the surgical instrument.

4. The robotic arm assembly of claim 1, wherein the instrument drive unit defines a longitudinal axis (L), and wherein the four motors are arranged in a circle about the longitudinal axis.

5. The robotic arm assembly of claim 4, wherein the motor housing and the four motors are configured rotate together about the longitudinal axis

6. The robotic arm assembly of Claim 6 wherein a first motor of the four motors defines a first motor axis, and wherein the first motor is configured to independently rotate about the first motor axis when the motor housing rotates about the longitudinal axis of the instrument drive unit.

7. The robotic arm assembly of claim 7, wherein the first motor axis of the first motor and the longitudinal axis of the instrument drive unit are parallel to one another.

8. The robotic arm assembly, wherein a first gear box assembly of the gear box assemblies includes a reduction gear assembly (118b) and a coupler (118c) secured to the reduction gear assembly, wherein the first motor includes a drive shaft (114a), wherein the reduction gear assembly of the first gear box assembly couples to the drive shaft of the first motor, and wherein the reduction gear assembly is configured to reduce an output from the drive shaft of the first motor to the coupler of the first gear box assembly.

## Patentansprüche

1. Roboterarmanordnung (20), umfassend:
einen Roboterarm (22, 24, 26);
eine Instrumentenantriebseinheit (50), die konfiguriert ist, um fünf Rotationsfreiheitsgrade bereitzustellen, die mit dem Roboterarm gekoppelt ist und einschließt:
ein Motorgehäuse (124);
Motoren (114), die in dem Motorgehäuse gelagert und mit dem Motorgehäuse rotierbar sind, wobei die Motoren vier Motoren einschließen, wobei jeder Motor einen der fünf Rotationsfreiheitsgrade der Instrumentenantriebseinheit bereitstellt;
eine Motorwicklung (132) und einen Magnet (128), die positioniert sind, um das Motorgehäuse zu umgeben, wobei die Motorwicklung konfiguriert ist, um mit dem Motorgehäuse relativ zu dem Magnet zu rotieren, um als ein Motor zu wirken und einen der fünf Rotationsfreiheitsgrade bereitzustellen; und
Getriebeanordnungen (118), die mit den Motoren gekoppelt und konfiguriert sind, um eine Antriebsleistung von den Motoren zu einem chirurgischen Instrument (30) zu übertragen.

2. Roboterarmanordnung nach Anspruch 1, ferner umfassend das chirurgische Instrument.

3. Roboterarmanordnung nach Anspruch 2, ferner umfassend ein steriles Schnittstellenmodul (80), das die Instrumentenantriebseinheit mit dem chirurgischen Instrument koppelt.

4. Roboterarmanordnung nach Anspruch 1, wobei die Instrumentenantriebseinheit eine Längsachse (L) definiert und wobei die vier Motoren in einem Kreis um die Längsachse herum angeordnet sind.

5. Roboterarmanordnung nach Anspruch 4, wobei das Motorgehäuse und die vier Motoren konfiguriert sind, um gemeinsam um die Längsachse herum zu rotieren

6. Roboterarmanordnung nach Anspruch 6, wobei ein erster Motor der vier Motoren eine erste Motorachse definiert und wobei der erste Motor konfiguriert ist, um um die erste Motorachse herum unabhängig zu rotieren, wenn das Motorgehäuse um die Längsachse der Instrumentenantriebseinheit herum rotiert.

7. Roboterarmanordnung nach Anspruch 7, wobei die erste Motorachse des ersten Motors und die Längsachse der Instrumentenantriebseinheit parallel zueinander sind.

8. Roboterarmanordnung, wobei eine erste Getriebeanordnung der Getriebeanordnungen eine Untersetzungsgetriebeanordnung (118b) und eine Kupplung (118c) einschließt, die an der Untersetzungsgetriebeanordnung befestigt ist, wobei der erste Motor eine Antriebswelle (114a) einschließt, wobei die Untersetzungsgetriebeanordnung der ersten Getriebeanordnung mit der Antriebswelle des ersten Motors gekoppelt ist und wobei die Untersetzungsgetriebeanordnung konfiguriert ist, um eine Ausgabe von der Antriebswelle des ersten Motors an die Kupplung der ersten Getriebeanordnung zu reduzieren.

## Revendications

1. Ensemble bras robotique (20), comprenant :
un bras robotique (22, 24, 26) ;
une unité d'entraînement d'instrument (50) conçue pour fournir cinq degrés de liberté de rotation accouplée au bras robotique et comportant :
un boîtier de moteur (124) ;
des moteurs (114) supportés dans le boîtier de moteur et pouvant tourner avec le boîtier de moteur, dans lequel les moteurs comportent quatre moteurs, chaque moteur fournissant l'un des cinq degrés de liberté de rotation de l'unité d'entraînement d'instrument ;
un enroulement de moteur (132) et un aimant (128) positionnés pour entourer le boîtier de moteur, l'enroulement de moteur étant conçu pour tourner avec le boîtier de moteur par rapport à l'aimant pour agir comme un moteur et fournir l'un des cinq degrés de liberté de rotation ; et
des ensembles de boîte d'engrenages (118) accouplés aux moteurs et conçus pour transmettre une puissance d'entraînement des moteurs à un instrument chirurgical (30).

2. Ensemble bras robotique selon la revendication 1, comprenant en outre l'instrument chirurgical.

3. Ensemble bras robotique selon la revendication 2, comprenant en outre un module d'interface stérile (80) qui accouple l'unité d'entraînement d'instrument à l'instrument chirurgical.

4. Ensemble bras robotique selon la revendication 1, dans lequel l'unité d'entraînement d'instrument définit un axe longitudinal (L), et dans lequel les quatre moteurs sont agencés en cercle autour de l'axe longitudinal.

5. Ensemble bras robotique selon la revendication 4, dans lequel le boîtier de moteur et les quatre moteurs sont conçus pour tourner ensemble autour de l'axe longitudinal

6. Ensemble bras robotique selon la revendication 6, dans lequel un premier moteur parmi les quatre moteurs définit un premier axe de moteur, et dans lequel le premier moteur est conçu pour tourner indépendamment autour du premier axe de moteur lorsque le boîtier de moteur tourne autour de l'axe longitudinal de l'unité d'entraînement d'instrument.

7. Ensemble bras robotique selon la revendication 7, dans lequel le premier axe de moteur du premier moteur et l'axe longitudinal de l'unité d'entraînement d'instrument sont parallèles l'un à l'autre.

8. Ensemble bras robotique, dans lequel un premier ensemble de boîte d'engrenages des ensembles de boîte d'engrenages comporte un ensemble démultiplicateur (118b) et un coupleur (118c) fixé à l'ensemble démultiplicateur, dans lequel le premier moteur comporte un arbre d'entraînement (114a), dans lequel l'ensemble démultiplicateur du premier ensemble de boîte d'engrenages s'accouple à l'arbre d'entraînement du premier moteur, et dans lequel l'ensemble démultiplicateur est conçu pour réduire une sortie de l'arbre d'entraînement du premier moteur vers le coupleur du premier ensemble de boîte d'engrenages.
